(19) Europäisches Patentamt
European Patent Office
Office européen des brevets

(11) Veröffentlichungsnummer: **0 268 088**
**B1**

(12) **EUROPÄISCHE PATENTSCHRIFT**

(45) Veröffentlichungstag der Patentschrift: **23.05.90**

(51) Int. Cl.[5]: **C07C 309/35**, C07C 309/36

(21) Anmeldenummer: **87115238.5**

(22) Anmeldetag: **17.10.87**

(54) **1-Aminomethylnaphthalin-6-sulfonsäure, ihre Herstellung und ihre Verwendung.**

(30) Priorität: **31.10.86 DE 3637138**

(43) Veröffentlichungstag der Anmeldung:
**25.05.88 Patentblatt 88/21**

(45) Bekanntmachung des Hinweises auf die Patenterteilung:
**23.05.90 Patentblatt 90/21**

(84) Benannte Vertragsstaaten:
**BE CH DE FR GB IT LI NL**

(56) Entgegenhaltungen:
**EP-A- 0 158 073**

(73) Patentinhaber: **BAYER AG,**
**D-5090 Leverkusen 1 Bayerwerk(DE)**

(72) Erfinder: **Hammerschmidt, Erich, Dr., Kicke 19,**
**D-5060 Bergisch-Gladbach 1(DE)**
Erfinder: **Blank, Heinz Ulrich, Dr., Am Geusfelde 35,**
**D-5068 Odenthal(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft die neue Verbindung 1-Aminomethylnaphthalin-6-sulfonsäure, ein Verfahren zu ihrer Herstellung und ihre Verwendung als Zwischenprodukt zur Herstellung von 6-Hydroxynaphthalin-1-carbonsäure.

6-Hydroxynaphthalin-1-carbonsäure ist ein wichtiges Zwischenprodukt für die Herstellung biologisch aktiver Verbindungen, z.B. des Pharmakons Tolrestat (siehe z.B. EP-A1-0 059 596). Außerdem wird 6-Hydroxynaphthalin-1-carbonsäure auch als Komponente für die Herstellung von Polyestern verwendet (siehe z.B. J.A.-Patent No. 134 778).

Im Hinblick auf die Bedeutung der 6-Hydroxynaphthalin-1-carbonsäure besteht daher großes technisches Interesse an einem wirtschaftlichen Verfahren zur Herstellung dieser Verbindung in technischem Maßstab.

Für die Herstellung von 6-Hydroxynaphthalin-1-carbonsäure sind bereits verschiedene Synthesewege bekannt; diese haben jedoch alle schwerwiegende Nachteile, vor allem, daß sie von teuren, in technischen Mengen z.T. noch gar nicht zugänglichen Ausgangsverbindungen ausgehen und/oder Reaktionen erfordern, die aus gewerbehygienischen und umwelttechnischen Gründen, wenn überhaupt, dann nur unter hohem apparativen Aufwand durchführbar sind und/oder zu schlechte Ausbeuten liefern.

Bei diesen bekannten Synthesewegen handelt es sich im einzelnen um folgende Verfahren:

Verfahren 1:

Es geht von der 1-Aminonaphthalin-6-sulfonsäure (Clevesäure 1.6) aus; diese wird diazotiert und nach Sandmeyer mit Alkalicyaniden in Gegenwart von Schwermetallen (z.B. Kupfer) in die 1-Cyannaphthalin-6-sulfonsäure umgewandelt. Diese wird durch Hydrolyse und nachfolgende Alkalischmelze in die 6-Hydroxynaphthalin-1-carbonsäure überführt (siehe Ullmanns Enzyklopädie der technischen Chemie 4. Auflage Bd. 17, S. 89 und die ausführliche Beschreibung dieses Syntheseweges in Soc. 1923, S. 1641-1645).

Die Nachteile dieses Verfahrens sind, daß die für die Reaktion benötigte reine, d.h. isomerenfreie Clevesäure-1.6 eine relativ teure Ausgangsverbindung ist und daß die Sandmeyer-Reaktion, wegen der Verwendung von Alkalicyaniden und der Schwermetalle einen hohen apparativen, verfahrens- und sicherheitstechnischen Aufwand erfordert.

Verfahren 2:

Es geht ebenfalls von reiner Clevesäure-1.6 aus; diese wird aber acyliert. Die auf diese Weise erhaltene 1-Acylaminonaphthalin-6-sulfonsäure wird dann in einer über mehrere Stufen verlaufenden Reaktion in das 1-Iod-6-methoxy-naphthalin umgewandelt und dieses in einer Grignard-Reaktion mit Magnesium und Kohlendioxid in die 6-Methoxynaphthalin-1-carbonsäure überführt (siehe B. 68, S. 2087ff (1936)).

Abgesehen davon, daß dieses Verfahren ebenfalls von der teuren Clevesäure-1.6 ausgeht, ist das Verfahren wegen seiner Umständlichkeit und der schwierigeren Durchführbarkeit einzelner Reaktionsstufen (z.B. der Grignard-Reaktion) für eine Herstellung der 6-Hydroxy-naphthalin-1-carbonsäure in technischem Maßstab ungeeignet.

Verfahren 3:

Es geht von der Naphthalin-1-carbonsäure aus; diese wird sulfoniert. Die aus dem bei der Sulfonierung anfallenden Isomerengemisch isolierte reine 1-Carboxy-naphthalin-6-sulfonsäure wird durch Alkalischmelze in die 6-Hydroxy-naphthalin-1-carbonsäure umgewandelt (siehe Soc. 1923, S. 1641-1647, insbesondere 1645-1646).

Die Nachteile dieses Verfahrens bestehen darin, daß die Naphthalin-1-carbonsäure teuer und in technischem Maßstab nur schwer erhältlich ist, ferner daß die Ausbeute an 1-Carboxynaphthalin-6-sulfonsäure in dem bei der Sulfonierung anfallenden Sulfonierungsgemisch niedrig ist und die Isolierung der 1-Carboxynaphthalin-6-sulfonsäure aus diesem Gemisch mit erheblichen Schwierigkeiten und weiteren Ausbeuteverlusten verbunden ist.

Verfahren 4:

Es geht vom 6-Alkoxytetralon aus; dieses wird über 6-Alkoxy-1-cyano-3,4-dihydronaphthalin und 6-Alkoxy-1-cyano-naphthalin in die 6-Alkoxy-naphthalin-1-carbonsäure überführt (siehe US-PS 4 590 010; J. Org. Chem. 1983, S. 5134-5135).

Die Nachteile dieses Verfahrens sind, daß es von einer teuren Spezialchemikalie, dem 6-Methoxy-tetralon, ausgeht und daß dessen Cyanierung in organischen Lösungsmitteln mit Alkalicyaniden in Gegenwart stöchiometrischer Mengen Lewis-Säure unter Verwendung von Phasentransferkatalysatoren oder - gemäß J. Org. Chem. 1983 loc. cit. - mit Trimethylsilylcyanid, Bortrifluorid, Pyridin und Phosphoroxichlorid aus gewerbehygienischen und umwelttechnischen Gründen in technischem Maßstab nicht durchführbar ist.

Verfahren 5:

Es geht von Anisol aus; dieses wird mit Furan-2-carbonsäure in Gegenwart eines großen Überschusses an Aluminiumtrichlorid zur 6-Methoxy-naphthalin-1-carbonsäure umgesetzt (siehe J. Am. Chem. Soc. 69, S. 2262 und EP-A1 200 840).

Das Verfahren ist wegen der in ihm zu verwendenden teuren Ausgangsverbindungen, der erforderlichen großen Aluminiumchloridmengen und der gleichzeitig mit ihm erreichten schlechten Ausbeuten für eine Darstellung von 6-Hydroxynaphthalin-1-carbonsäure in technischem Maßstab ungeeignet.

Es wurde nun gefunden, daß 6-Hydroxynaphthalin-1-carbonsäure aus einem billigen und in technischen Mengen zur Verfügung stehenden Ausgangsmaterial auf einem technisch ohne Schwierigkeiten durchführbaren Syntheseweg erhalten werden kann, wenn man von Naphthalin-2-sulfonsäure ausgeht, diese in an sich bekannter Weise amidomethyliert, die Amidomethylierungsprodukte verseift, die nach dieser Verseifung anfallende 1-Aminomethylnaphthalin-6-sulfonsäure in an sich bekannter Weise zur 1-Carboxynaphthalin-6-sulfonsäure oxidiert und diese durch Alkalischmelze in 6-Hydroxynaphthalin-1-carbonsäure überführt.

Überraschenderweise wurde gefunden, daß bei der Hydrolyse des bei der Amidoalkylierung von Naphthalin-2-sulfonsäure anfallenden, Isomere und Nebenprodukte enthaltenden Reaktionsgemisches unmittelbar reine, praktisch isomerenfreie 1-Aminomethylnaphthalin-6-sulfonsäure erhalten wird.

Die 1-Aminomethylnaphthalin-6-sulfonsäure und deren Herstellung sind neu. Dadurch, daß die 1-Aminomethylnaphthalin-6-sulfonsäure in einfacher Weise ohne Schwierigkeiten in reiner Form aus Naphthalin-2-sulfonsäure erhältlich ist und sich in einer auch in technischem Maßstab ohne Schwierigkeiten durchführbaren Reaktionsfolge in die gewünschte 6-Hydroxynaphthalin-1-carbonsäure umwandeln läßt, eröffnet sie einen neuen, interessanten Syntheseweg zur Herstellung von 6-Hydroxynaphthalin-1-carbonsäure aus Naphthalin-2-sulfonsäure.

Die Erfindung betrifft daher die neue Verbindung 1-Aminomethylnaphthalin-6-sulfonsäure

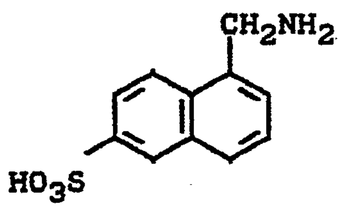

(I),

ein Verfahren zu deren Herstellung und deren Verwendung zur Herstellung von 6-Hydroxynaphthalin-1-carbonsäure.

Das erfindungsgemäße Verfahren zur Herstellung der 1-Aminomethylnaphthalin-6-sulfonsäure ist dadurch gekennzeichnet, daß man Naphthalin-2-sulfonsäure in an sich bekannter Weise in saurem Medium mit Verbindungen der Formel

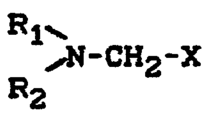

(II),

in der

$R_1$ für einen Acylrest und

$R_2$ für einen Acylrest oder, vorzugsweise, ein Wasserstoffatom, stehen oder $R_1$ und $R_2$ zusammen einen Diacylrest bilden,

X für ein Halogenatom, eine $R_3O$- oder eine $(R_1)(R_2)N$-Gruppe und

$R_3$ für einen Acyl- oder einen $(R_1)(R_2)N-CH_2$-Rest oder, vorzugsweise, ein Wasserstoffatom stehen,

amidoalkyliert und anschließend aus dem Amidoalkylierungsprodukt den Acylrest $R_1$ bzw. die Acylreste $R_1$ und $R_2$ durch saure oder alkalische Hydrolyse abspaltet.

Die erfindungsgemäße Amidoalkylierung der Naphthalin-2-sulfonsäure mit den Verbindungen der Formel (II) wird in für die Amidoalkylierung aromatischer Verbindungen an sich bekannter Weise vorgenommen; solche Amidoalkylierungen aromatischer Verbindungen sind z.B. in Org. Reactions 14, S. 52 u.f.;

Synthesis 1970, S. 49 u.f. und Synthesis 1984, S. 85 u.f., ferner in der DE-OS 2 264 698 und 3 408 300 beschrieben. Naphthalin-2-sulfonsäure und Amidoalkylierungsmittel werden in einem Molverhältnis von etwa 1:0,6 bis 1:1,4 bei Temperaturen von 0 bis 100°C vorzugsweise in 60 bis 100 gew.-%iger Schwefelsäure umgesetzt.

Die Naphthalin-2-sulfonsäure kann in Form der freien Säure oder in Form ihrer Salze eingesetzt werden. Sie kann in reiner Form aber auch im Gemisch mit anderen Naphthalin-mono- und -disulfonsäuren eingesetzt werden.

Bevorzugt wird Naphthalin-2-sulfonsäure als Natriumsalz oder in Form von Sulfonierungsgemischen eingesetzt, wie sie bei der Umsetzung von 1 Mol Naphthalin mit 0,8 bis 2,0 Mol 98-100 %iger Schwefelsäure bei Temperaturen von 100 bis 170°C anfallen. Der Gehalt dieser Sulfonierungsgemische an Naphthalin-2-sulfonsäure beträgt im allgemeinen 40 bis 70 Gew.-%.

Als Amidoalkylierungsmittel der Formel (II) kommen die verschiedenartigsten Amidoalkylierungsmittel in Betracht. Als Acylreste seien für $R_1$ und $R_2$ die Reste von aliphatischen und aromatischen Carbon- und Sulfonsäuren und für $R_3$ die Reste von aliphatischen und aromatischen Carbonsäuren genannt. Als Acylreste aliphatischer Carbonsäuren seien beispielsweise genannt: gegebenenfalls durch Chloratome substituierte $C_1$-$C_4$-Alkanoyl oder $C_1$-$C_4$-Alkenoyl-Reste wie der Formyl-, Acetyl-, Propionyl-, Chloracetyl-oder Acryloylrest; als Reste aromatischer Carbonsäuren seien beispielsweise der Benzoyl-, der Isophthaloyl- oder der Terephthaloylrest genannt. Als Reste aliphatischer Sulfonsäuren sei beispielsweise der Methansulfonylrest, und als Beispiel für Reste aromatischer Sulfonsäuren der Benzolsulfonyl-und der p-Toluolsulfonylrest genannt. $R_1$ und $R_2$ können ferner zusammen einen Diacylrest einer gesättigten oder ungesättigten aliphatischen Dicarbonsäure oder einer aromatischen o-Dicarbonsäure bilden. Als Reste solcher Dicarbonsäuren seien beispielsweise genannt die Reste der Adipin-, Malein- und der Phthalsäure.

Bevorzugt sind solche Verbindungen der Formel (II), in der

$R_1$für einen gegebenenfalls durch Chlor substituierten $C_1$-$C_4$-Alkanoyl- oder $C_1$-$C_4$-Alkenoyl-Rest oder den Rest einer aromatischen Carbonsäure steht,

$R_2$Wasserstoff ist, oder

$R_1$ und $R_2$ zusammen den Diacylrest einer gesättigten oder ungesättigten aliphatischen Dicarbonsäure oder einer aromatischen o-Dicarbonsäure bilden und

Xfür Chlor, eine Hydroxy- oder eine $R_1$NH-Gruppe steht.

Besonders bevorzugte Amidoalkylierungsmittel der Formel (II) sind N-Hydroxylmethylacetamid, N-Hydroxymethylchloracetamid, N-Hydroxymethylbenzamid, N-Hydroxymethylphthalimid, N,N'-(Bishydroxymethyl)-isophthalsäure- und -terephthalsäurediamid und Bis-benzamidomethan.

Die N-Hydroxymethylgruppen aufweisenden Amidoalkylierungsmittel können in Form reiner Verbindungen aber auch in Form von Reaktionsprodukten eingesetzt werden, wie sie bei der Umsetzung der entsprechenden Amide mit stö chiometrischen Mengen Formaldehyd oder Paraformaldehyd anfallen. Bei der Umsetzung der Amide mit wäßrigen Formaldehydlösungen werden Lösungen oder Suspensionen der Reaktionsprodukte in Wasser, bei der Umsetzung mit Paraformaldehyd feste oder flüssige Reaktionsprodukte erhalten. Diese flüssigen oder festen Reaktionsprodukte oder deren wäßrige Lösungen und Suspensionen können unmittelbar für die Amidoalkylierungsreaktion verwendet werden.

Die Hydrolyse der 1-N-Acylamido- bzw. 1-N-Acylimidomethylnaphthalin-6-sulfonsäuren zur 1-Aminomethylnaphthalin-6-sulfonsäure wird in für die Abspaltung von Acylgruppen aus Amidogruppen an sich bekannter Weise vorgenommen. (s. DE-OS 2 264 6 98 und 3 408 300 und Houben-Weyl Bd. 11/1, S. 927-931).

Nach beendeter Hydrolyse wird der pH-Wert des alkalischen Hydrolysegemisches durch Zugabe von Säure auf einen Wert ≤αμρ˜ 6,5, vorzugsweise ≤ αμρ˜ 6, eingestellt. Anschließend wird die sich als Niederschlag abscheidende freie 1-Aminomethylnaphthalin-6-sulfonsäure mechanisch, z.B. durch Abfiltrieren oder Absaugen oder Zentrifugieren, abgetrennt. Im Falle einer sauren Hydrolyse fällt die freie 1-Aminomethylnaphthalin-6-sulfonsäure unmittelbar nach ihrer Bildung aus dem Reaktionsgemisch aus und wird aus diesem isoliert.

Die 1-Aminomethylnaphthalin-6-sulfonsäure kann in an sich bekannter Weise nach Sommelet (siehe z.B. Houben Weyl, Methoden der Organischen Chemie, 4. Aufl., Bd. 7/1 S. 198 oder Organic Reactions 8, 197 (1954)), d.h. durch Umsetzung mit Hexamethylentetramin in die 1-Formylnaph thalin-6-sulfonsäure überführt werden. Die 1-Formylnaphthalin-6-sulfonsäure wird ihrerseits nach für die Oxidation von Aldehyden zu Carbonsäuren bekannten Verfahren (siehe z.B. Houben Weyl loc. cit. Bd. 8, S. 407-413) z.B. durch Oxidation mit Permanganat (siehe z.B. Houben Weyl Bd. 4 l/b, S. 615-621) zur 1-Carboxynaphthalin-6-sulfonsäure oxidiert.

Nach einem anderen Verfahren wird die 1-Aminomethylnaphthalin-6-sulfonsäure in an sich bekannter Weise mittels Dichromat- oder Chromatsalzen im neutralen bis alkalischen Milieu unmittelbar zur 1-Carboxynaphthalin-6-sulfonsäure oxidiert (siehe z.B. Houben-Weyl. Bd. 4/1B, S. 435).

Beispiel 1

In 1.032 g vorgelegte 95 gew.-%ige Schwefelsäure werden innerhalb einer Stunde unter Rühren bei 15 bis 20°C gleichzeitig 230 g (1 Mol) Naphthalin-2-sulfonsäure (Natriumsalz) und 151 g (1 Mol) N-Hydroxymethylbenzamid eingetragen. Das Reaktionsgemisch wird 3 Stunden bei 20 bis 25°C gerührt und anschließend in 4.160 g 25 %ige Natronlauge eingerührt. Die entstandene heiße alkalische Suspension wird zum Verseifen der Benzamidomethylverbindung 4 Stunden im Autoklaven auf 140°C erwärmt. Anschließend wird die heiße alkalische Reaktionslösung auf einen pH-Wert von 6,0 angesäuert; nach dem Abkühlen auf 35°C wird die als inneres Salz schwer lösliche 1-Aminomethylnaphthalin-6-sulfonsäure abfiltriert und mit Wasser gewaschen.

Nach dem Trocknen werden 149,5 g (= 60 % der Theorie) 1-Aminomethylnaphthalin-6-sulfonsäure in Form eines elfenbeinfarbenen Kristallisats erhalten. Reinheitsgrad der Säure: 95,2 %. Das Produkt enthält noch 0,3 Gew.-% Isomere, 0,6 Gew.-% Wasser und 3,9 Gew.-% Natriumsulfat.
NMR-Spektrum der 1-Aminomethylnaphthalin-6-sulfonsäure (NaOD): δ 3,75 (s, 2H), 7,10 (d, 1H), 7,19 (m, 1H), 7,49 (d, 1H), 7,63-7,67 (m, 2H), 8,08 (s, 1H).

Summenformel $C_{11}H_{11}NO_3S$
Elementaranalyse (der 100 %igen Verbindung):
berechnet: C 55,68 %, H 4,67 %, N 5,90 %
gefunden : C 55,60 %, H 4,72 %, N 5,94 %.

Beispiel 2

300,4 g Sulfonierungsgemisch, das bei der Sulfonierung von Naphthalin mit Schwefelsäure (100 %ig) erhalten wurde und folgende Zusammensetzung aufwies (%-Angaben = Gewichts-%)

0,51 % Naphthalin-2,6-disulfonsäure
0,25 % Naphthalin-1,5-disulfonsäure
1,47 % Naphthalin-2,7-disulfonsäure
3,77 % Naphthalin-1,6-disulfonsäure
2,52 % Naphthalin-1,7-disulfonsäure
0,52 % Naphthalin-1,3-disulfonsäure
3,37 % Naphthalin-1-sulfonsäure
65,86 % Naphthalin-2-sulfonsäure
0,20 % Naphthalin
0,10 % Dinaphthylsulfone
14,5 % $H_2SO_4$
6,9 % $H_2O$

werden in 353,4 g 100 %iger Schwefelsäure bei 10-15°C gelöst. In die Lösung werden innerhalb von 5 Stunden bei 15-20°C 98,9 g N-Hydroxymethylchloracetamid (Gehalt 99,9 %) gleichmäßig eingetragen. Das Reaktionsgemisch wird 5 Stunden bei 20°C gerührt. Anschließend wird es mit 915 g Wasser verdünnt und 3h auf 105-110°C erhitzt. Bereits nach 30 min. beginnt die 1-Aminomethylnaphthalin-6-sulfonsäuree auszufallen. Die Suspension wird auf 20°C abgekühlt und die 1-Aminonaphthalin-6-sulfonsäure abgetrennt. Sie wird mit 100 ml Wasser gewaschen und anschließend getrocknet.

Ausbeute 99,5 g (= 49 % der Theorie bezogen auf N-Hydroxymethylchloracetamid). Reinheitsgrad der Säure: 93,5 %. Das Produkt enthält noch 1,6 % Isomere, 3,1 % Schwefelsäure und 1,8 % Wasser.

Beispiel 3

(Weiterverarbeitung der 1-Aminomethylnaphthalin-6-sulfonsäure zur 6-Hydroxynaphthalin-1-carbonsäure)

a) Oxidation zur 1-Carboxynaphthalin-6-sulfonsäure
α) 118,6 g (0,5 Mol) 1-Aminomethylnaphthalin-6-sulfonsäure (100 %) werden in 450 ml 50 %iger Essigsäure suspendiert und mit 140 g (1,0 Mol) Hexamethylentetramin versetzt und auf Rückflußtemperatur erhitzt. Nach 4 Stunden wird die klare Lösung mit 200 ml konzentrierter Salzsäure versetzt und eine weitere Stunde auf Rückflußtemperatur erhitzt. Anschließend wird überschüssiger Formaldehyd zusammen mit Essigsäure und Wasser abdestilliert, wobei aber das ursprüngliche Volumen der Reaktionsmischung durch Wasserzugabe konstant gehalten wird. Sobald kein Formaldehyd im Destillat mehr nachweisbar ist, wird das siedende Reaktiongemisch mit 50 %iger wäßriger Natronlauge auf einen pH-Wert von 8 eingestellt und mit 110 g (0,7 Mol) Kaliumpermanganat versetzt. Das Reaktionsgemisch wird 1 Stunde auf Rückflußtemperatur erhitzt. Nach dem Abtrennen des Mangandioxids wird die Reaktionslösung mit konzentrierter Salzsäure auf einen pH-Wert von 3 eingestellt. Nach dem Abkühlen des Reaktionsgemisches auf 20°C wird die in Form ihres Natriumsalzes ausgefallene 1-Carboxynaphthalin-6-sulfonsäure abfiltriert.

Ausbeute: 101 g (= 80 % der Theorie) 1-Carboxynaphthalin-6-sulfonsäure (100 %ig).

β) 118,6 g (0,5 Mol) 1-Aminomethylnaphthalin-6-sulfonsäure (100 %) werden mit 375 ml Wasser und ca. 50 g NaOH 45 % verrührt; der pH-Wert des Gemisches wird auf 7-7,5 eingestellt. Nach Zusatz von 149 g Natriumdichromat (0,5 Mol) wird die Mischung im Autoklaven 15 h auf 240°C erhitzt. Nach dem Abkühlen wird der grüne Chromhydroxid-Rückstand abfiltriert und mit heißem Wasser gewaschen. Das Filtrat wird in der Hitze mit Salzsäure (30 %ig) auf pH 2 angesäuert und anschließend auf 20°C abgekühlt. Die in Form ihres Natriumsalzes ausfallende 1-Carboxynaphthalin-6-sulfonsäure wird abfiltriert und mit 100 ml ges. NaCl-Lösung gewaschen.

Ausbeute: 120 g (= 95 % der Theorie) 1-Carboxynaphthalin-6-sulfonsäure (100 %ig).

b) Die 1-Carboxynaphthalin-6-sulfonsäure wird nach der in J. Chem. Soc. 1923, 164 ff. beschriebenen Arbeitsweise durch Alkalischmelze in die 6-Hydroxynaphthalin-1-carbonsäure überführt.

**Patentansprüche**

1. 1-Aminomethylnaphthalin-6-sulfonsäure

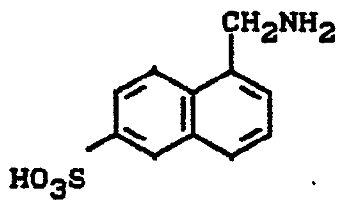

(I),

2. Verfahren zur Herstellung von 1-Aminomethyl-naphthalin-6-sulfonsäure, dadurch gekennzeichnet, daß man Naphthalin-2-sulfonsäure in an sich bekannter Weise in saurem Medium mit Verbindungen der Formel

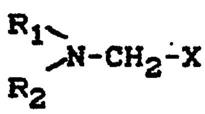

(II),

in der

$R_1$ für einen Acylrest,

$R_2$ für ein Wasserstoffatom oder einen Acylrest stehen oder $R_1$ und $R_2$ zusammen einen Diacylrest bilden,

X für ein Halogenaton, eine $R_3O$- oder eine $(R_1)(R_2)N$-Gruppe und

$R_3$ für ein Wasserstoffatom, eine Acyl- oder einen $(R_1)(R_2)N-CH_2$-Rest stehen,

amidoalkyliert und anschließend aus dem Amidoalkylierungsprodukt den Acylrest $R_1$ oder die Acylreste $R_1$ und $R_2$ durch saure oder alkalische Hydrolyse abspaltet.

3. Verfahren nach Anspruch 2 dadurch gekennzeichnet, daß man die Amidoalkylierung in 60 bis 100 gew.-%iger Schwefelsäure vornimmt.

4. Verfahren nach Anspruch 2 oder 3, dadurch gekennzeichnet, daß man Verbindungen der in Anspruch 2 angegebenen Formel (II) verwendet, in der

$R_1$ für einen gegebenenfalls durch Chlor substituierten $C_1$-$C_4$-Alkanoyl oder $C_1$-$C_4$-Alkenoyl-Rest oder den Rest einer aromatischen Carbonsäure steht,

$R_2$ Wasserstoff ist oder

$R_1$ und $R_2$ zusammen den Diacylrest einer gesättigten oder ungesättigten aliphatischen Dicarbonsäure oder einer aromatischen o-Dicarbonsäure bilden und

X für Chlor, eine Hydroxy- oder eine $R_1NH$-Gruppe steht.

5. Verfahren nach einem der Ansprüche 2, 3 oder 4, dadurch gekennzeichnet, daß man nach der Hydrolyse des Amidoalkylierungsproduktes die 1-Aminomethylnaphthalin-6-sulfonsäure bei pH ≤αμρ¨ 6,5 abtrennt.

6. Verwendung der 1-Aminomethylnaphthalin-6-sulfonsäure zur Herstellung von 6-Hydroxynaphthalin-1-carbonsäure.

**Claims**

1. 1-Aminomethylnaphtalene-6-sulphonic acid

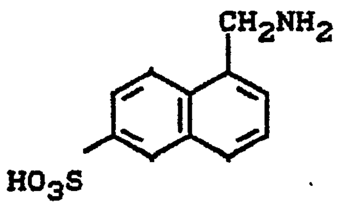

(I),

2. Process for the preparation of 1-aminomethylnaphthalene-6-sulphonic acid, characterized in that naphthalene-2-sulphonic acid is amidoalkylated in a manner which is known per se in an acid medium with compounds of the formula

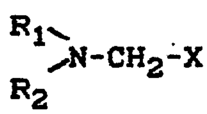

(II),

in which
$R_1$ represents an acyl radical,
$R_2$ represents a hydrogen atom or an acyl radical or $R_1$ and $R_2$ together form a diacyl radical,
X represents a halogen atom, an $R_3O$-group or an $(R_1)(R_2)N$–group and
$R_3$ represents a hydrogen atom, an acyl radical or an $(R_1)(R_2)N–CH_2$–radical,
and the acyl radical $R_1$ or the acyl radicals $R_1$ and $R_2$ are then split off from the amidoalkylation product by acid or alkaline hydrolysis.

3. Process according to Claim 2, characterized in that the amidoalkylation is carried out in 60 to 100% strength by weight sulphuric acid.

4. Process according to Claim 2 or 3, characterized in that compounds of the formula (II) given in Claim 2, in which
$R_1$ represents a $C_1$–$C_4$-alkanoyl or $C_1$–$C_4$-alkenoyl radical which is optionally substituted by chlorine, or the radical of an aromatic carboxylic acid,
$R_2$ is hydrogen or
$R_1$ and $R_2$ together form the diacyl radical of a saturated or unsaturated aliphatic dicarboxylic acid or of an aromatic o-dicarboxylic acid and X represents chlorine or a hydroxyl or an $R_1NH$ group,
are used.

5. Process according to one of Claims 2, 3 or 4, characterized in that after the hydrolysis of the amidoalkylation product, the 1-aminomethylnaphthalene-6-sulphonic acid is separated off at pH $\leq$ 6.5.

6. Use of 1-aminomethylnaphthalene-6-sulphonic acid for the preparation of 6-hydroxynaphthalene-1-carboxylic acid.

**Revendications**

1. L'acide 1-aminométhylnaphtalène-6-sulfonique

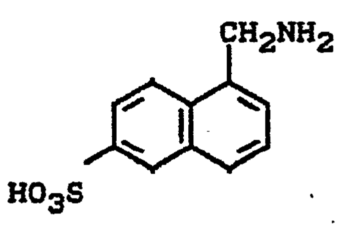

(I),

2. Procédé de préparation de l'acide 1-aminométhylnaphtalène-6-sulfonique, caractérisé en ce qu'on effectue l'amido-alkylation de l'acide naphtalène-2-sulfonique d'une manière connue en milieu acide avec des composés de formule

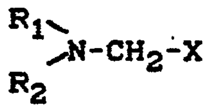

(II),

dans laquelle
$R_1$ représente un reste acyle,
$R_2$ est un atome d'hydrogène ou un reste acyle, ou bien $R_1$ et $R_2$ forment conjointement un reste diacyle,
X est un atome d'halogène, un groupe $R_3O-$ ou un groupe $(R_1)(R_2)N-$ et
$R_3$ représente un atome d'hydrogène, un reste acyle ou un reste $(R_1)(R_2)N-CH_2-$,
puis on élimine du produit d'amido-alkylation le reste acyle $R_1$ ou les restes acyle $R_1$ et $R_2$ par hydrolyse acide ou alcaline.

3. Procédé suivant la revendication 2, caractérisé en ce qu'on effectue l'amido-alkylation dans de l'acide sulfurique à 60–100 % en poids.

4. Procédé suivant la revendication 2 ou 3, caractérisé en ce qu'on utilise des composés de la formule (II) indiquée dans la revendication 2, dans laquelle
$R_1$ représente un reste alcanoyle en $C_1$ à $C_4$ ou alcénoyle en $C_1$ à $C_4$ substitué le cas échéant par du chlore, ou le reste d'un acide carboxylique aromatique
$R_2$ est l'hydrogène, ou bien
$R_1$ et $R_2$ ferment conjointement le reste diacyle d'un acide dicarboxylique aliphatique saturé ou non saturé ou d'un acide o-diacarboxylique aromatique et
X représente le chlore, un groupe hydroxy ou un groupe $R_1NH-$.

5. Procédé suivant l'une des revendications 2, 3 ou 4, caractérisé en ce qu'on sépare l'acide 1-aminométhylnaphtalène-6-sulfonique à un pH inférieur ou égal à 6,5 après l'hydrolyse du produit d'amido-alkylation.

6. Utilisation de l'acide 1-aminométhylnaphtalène-6-sulfonique pour la production de l'acide 6-hydroxynaphtalène-1-carboxylique.